# EUROPEAN PATENT APPLICATION

(11) **EP 3 664 100 A1**
(43) Date of publication of application: **10.06.2020**
(21) Application number: 18210451.3
(22) Date of filing: 05.12.2018
(51) Int. Cl.: G16H 40/60

(54) **ASSOCIATING NON-VERBAL COMMUNICATION CONTENT WITH VERBAL COMMUNICATION CONTENT**

(71) Applicant: Koninklijke Philips N.V., 5656 AG Eindhoven (NL)
(72) Inventor: PAWAR, Pravin, 5656 AE Eindhoven (NL); VAN GENUGTEN, Lenneke, 5656 AE Eindhoven (NL); SISODIA, Rajendra Singh, 5656 AE Eindhoven (NL); PADUKONE, Rishab, 5656 AE Eindhoven (NL); MOORKAN, Thasneem, 5656 AE Eindhoven (NL); DOTSCH, Ron, 5656 AE Eindhoven (NL)
(74) Representative: de Haan, Poul Erik

(57) **Abstract**

According to various embodiments, the invention relates an apparatus (100) for associating non-verbal communication content with verbal communication content, the apparatus comprising a processor (102) configured to obtain a portion of verbal communication content to be presented to a user; provide the portion of verbal communication content as an input to an intent determination model; determine, using the intent determination model, at least one intent of the portion of verbal communication content; determine, for the at least one intent, corresponding non-verbal communication content; and associate the corresponding non-verbal communication content with the portion of verbal communication content. The invention also relates to a method and a computer program product.

## Description

### FIELD OF THE INVENTION

The invention relates to non-verbal communication content and, more particularly, to an apparatus and method for associating non-verbal communication content with verbal communication content.

### BACKGROUND OF THE INVENTION

It is known in various industries and sectors to generate verbal communication content to be communicated to a user. The communication content, such as words, phrases, sentences or portions of an entire dialogue, may be presented to a user via a computing device, and the communication content may be delivered by the computing device, or a component thereof, rather than by a human. For example, verbal communication content may be delivered by a conversational agent. A conversational agent, or dialogue system, is a computer-based system intended to converse with a human user in a coherent manner. An effective conversational agent may give the impression to the user that the user is communicating with a human, rather than with a computer-based system (e.g. a cartoon character or other fictive person).

One field in which verbal communication content may be delivered to a user (e.g. via a conversational agent) is in a clinical setting, where a user may communicate with a virtual health coach or virtual health management coach in order to obtain advice, motivation and/or instructions regarding health management or improvement.

When verbal communication content is delivered by a human health management coach, the recipient of the communication content (i.e. the person with whom the human health management coach is conversing, such as a patient) is able to gain a deeper understanding of what the human health management coach is saying, as the patient is able to see the coach's body language and facial expressions, and is able to pick up on other patterns, such as inflections in the coach's voice, that may provide a deeper understanding about what they are saying. However, when verbal communication content is presented by a computer-based system, many of the additional conversation patterns are not conveyed and, as a result, the message being delivered to the patient may not be as effective.

Therefore, it would be useful to be able to provide a fuller communication experience to a user, which enables a user to gain a deeper understanding of the verbal communication content being presented to them. More generally, it would be useful to have a system that addresses one or more of the above-identified problems of existing systems.

### SUMMARY OF THE INVENTION

It has been recognized that an improved conversational experience may be provided to the user if, in addition to verbal communication content, non-verbal communication content is also delivered. Specifically, verbal communication content may be more effective if it is associated with corresponding non-verbal communication content, such as non-verbal aspects that might be exhibited by the human, but not necessarily by a computer-based system. According to embodiments disclosed herein, non-verbal communication content may be associated with corresponding verbal communication content. The verbal communication content may, in some embodiments, then be supplemented by the non-verbal communication content, when it is delivered to a user.

According to a first aspect, various embodiments disclosed herein provide an apparatus for associating non-verbal communication content with verbal communication content, the apparatus comprising a processor configured to obtain a portion of verbal communication content to be presented to a user; provide the portion of verbal communication content as an input to an intent determination model; determine, using the intent determination model, at least one intent of the portion of verbal communication content; determine, for the at least one intent, corresponding non-verbal communication content; and associate the corresponding non-verbal communication content with the portion of verbal communication content.

In some embodiments, the processor may be further configured to store the association between the corresponding non-verbal communication content with the portion of verbal communication content in a storage medium.

The processor may, in some embodiments, be further configured to deliver the non-verbal communication content and the portion of verbal communication content for presentation to the user.

In some embodiments, the non-verbal communication content may be communicated to the user by a conversational agent. The non-verbal communication content and the portion of verbal communication content to be presented to the user may be determined based on at least one previous interaction between the conversational agent and a user.

In some embodiments, the non-verbal communication content may comprise at least one of: a visual focus of attention, a facial expression, a head pose, a physical movement, a posture, a gesture, a mannerism, a voice modulation or inflection, and a paralanguage element.

The intent determination model may comprise a machine learning model trained to determine one or more intents from a portion of verbal communication content.

The processor may, in some embodiments, be configured to obtain the portion of verbal communication content from at least one of: a database; and a user input interface.

The processor may be further configured to receive an annotated recording of an interaction between a first human and a second human, wherein the annotated recording comprises an indication of at least one intent associated with a portion of the interaction. The processor may be further configured to train a classifier using the portion of the interaction and the at least one intent, wherein the trained classifier comprises the intent determination model.

According to a second aspect, various embodiments disclosed herein provide a method for associating non-verbal communication content with verbal communication content, the method comprising obtaining a portion of verbal communication content to be presented to a user; providing the portion of verbal communication content as an input to an intent determination model; determining, using the intent determination model, at least one intent of the portion of verbal communication content; determining, for the at least one intent, corresponding non-verbal communication content; and associating the corresponding non-verbal communication content with the portion of verbal communication content.

In some embodiments, the method may further comprise at least one of storing the association between the corresponding non-verbal communication content with the portion of verbal communication content in a storage medium; and presenting the non-verbal communication content and the portion of verbal communication content to the user.

Said presenting may comprise presenting the non-verbal communication content and the portion of verbal communication content to the user via a conversational agent.

In some embodiments, presenting the non-verbal communication content via a conversational agent may comprise causing the conversational agent to exhibit non-verbal communication characteristics and/or non-verbal communication patterns based on the non-verbal communication content.

The method may, in some embodiments, further comprise, prior to said obtaining, training an intent determination model by receiving an annotated recording of an interaction between a human coach and a subject, wherein the annotated recording comprises an indication of at least one intent associated with a portion of the interaction; and training the intent determination model using the portion of the interaction and the at least one intent.

According to a third aspect, various embodiments disclosed herein provide a computer program product comprising a non-transitory computer-readable medium, the computer-readable medium having computer-readable code embodied therein, the computer-readable code being configured such that, on execution by a suitable computer or processor, the computer or processor is caused to perform steps of the methods disclosed herein.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematic illustration of an example of an apparatus according to various embodiments;
Fig. 2 is a flowchart of an example of a method of associating non-verbal communication content with verbal communication content according to various embodiments;
Fig. 3 is an illustration of an example of applying non-verbal communication content to an avatar:
   Fig. 4 is a flowchart of an example of a method of analyzing a human coaching session;
   Fig. 5 is a flowchart of an example of a method of training a classifier to perform as an intent determination model;
   Fig. 6 is a flowchart of a further example of associating non-verbal communication content with verbal communication content;
   Fig. 7 is a flowchart of a further example of associating non-verbal communication content with verbal communication content; and
   Fig. 8 is a schematic illustration of a processor in communication with a computer-readable medium.

### DETAILED DESCRIPTION OF EMBODIMENTS

According to embodiments disclosed herein, non-verbal communication content (also referred to as non-verbal content) may be associated with verbal communication content (also referred to as verbal content) that is to be communicated to a user. The expression "verbal communication content" as used herein is intended to include verbal or textual elements (e.g. words) of a conversation or dialogue. The verbal communication content may then be enhanced and supplemented by the non-verbal communication content, thereby adding context, emotion, and/or understanding to the communication content to be delivered to a user. More generally, by supplementing the verbal content with non-verbal content, the verbal content may be provided with intent (i.e. the intentions of the communicator or intended meaning of the verbal content to be delivered may be expressed along with the verbal content).

Some aspects of the invention disclosed herein are described in the context of a clinical setting, wherein the content (e.g. the non-verbal content and/or the verbal content) comprises health coaching content to be delivered, for example, by a health management coach, or health coach, to a user, such as a patient. However, it will be apparent that embodiments disclosed herein may be applied in fields other than the medical field. In general, non-verbal content may be associated with verbal content, and the content may be used for some other purpose, such as for communication to any user. For example, the content may be delivered by a conversational agent representing a customer service adviser to a customer.

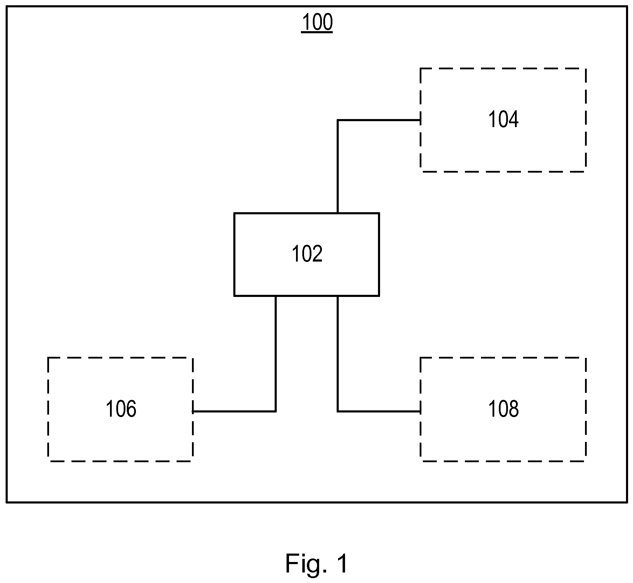

According to a first aspect, embodiments disclosed herein provide an apparatus for associating non-verbal communication content with verbal communication content. Referring to the drawings, Fig. 1 shows a block diagram of such an apparatus 100 that can be used for associating non-verbal content with verbal content. With reference to Fig. 1, the apparatus 100 comprises a processor 102 that controls the operation of the apparatus 100 and that can implement the methods described herein. The apparatus 100 may further comprise a memory 106 comprising instruction data representing a set of instructions. The memory 106 may be configured to store the instruction data in the form of program code that can be executed by the processor 102 to perform the method described herein. In some implementations, the instruction data can comprise a plurality of software and/or hardware modules that are each configured to perform, or are for performing, individual or multiple steps of the method described herein. In some embodiments, the memory 106 may be part of a device that also comprises one or more other components of the apparatus 100 (for example, the processor 102 and/or one or more other components of the apparatus 100). In alternative embodiments, the memory 106 may be part of a separate device to the other components of the apparatus 100. For example, the apparatus 100 may be implemented as part of a cloud computing environment.

The processor 102 of the apparatus 100 can be configured to communicate with the memory 106 to execute the set of instructions. The set of instructions, when executed by the processor may cause the processor to perform steps of the methods described herein. The processor 102 can comprise one or more processors, processing units, multi-core processors or modules that are configured or programmed to control the apparatus 100 in the manner described herein. In some implementations, for example, the processor 102 may comprise a plurality of processors, processing units, multi-core processors and/or modules configured for distributed processing. It will be appreciated by a person skilled in the art that such processors, processing units, multi-core processors and/or modules may be located in different locations and may each perform different steps and/or different parts of a single step of the methods described herein.

In some embodiments, as illustrated in Fig. 1, the apparatus 100 may comprise at least one user interface 104 configured to receive any of the user inputs described herein. The user interface 104 may allow a user of the apparatus 100 to manually enter instructions, data, or information relating to the method described herein. For example, a user interface 104 may be used by a user (e.g. a patient) to view, listen to or otherwise receive communication content (e.g. verbal and non-verbal content). In some embodiments, the user interface 104 may enable a user to input communication content (e.g. the user's part of a conversation or dialogue), for example in response to communication content delivered by the apparatus to the user (e.g. a conversational agent's part of a conversation or dialogue). The user interface 104 may be any type of user interface that enables a user of the apparatus 100 to provide a user input, interact with and/or control the apparatus 100. For example, the user interface 104 may comprise one or more switches, one or more buttons, a keypad, a keyboard, a mouse, a touch screen, a microphone or an application (for example, on a tablet or smartphone), or any other user interface, or combination of user interfaces that enables the user to provide data to the apparatus and/or via which the user can consume information from the apparatus 100.

In some embodiments, the user interface 104 (or another user interface of the apparatus 100) may enable rendering (or output or display) of information, data or signals to a user of the apparatus 100. As such, a user interface 104 may be for use in providing a user of the apparatus 100 (e.g. a customer or a patient) with information relating to or resulting from the method according to embodiments herein. The processor 102 may be configured to control one or more user interfaces 104 to provide information resulting from the method according to embodiments described herein. For example, the processor 102 may be configured to control one or more user interfaces 104 to render (or output or display) data (e.g. communication content, such as a line of a dialogue) using the methods described herein and/or any other outputs of the methods described herein. The user interface 104 may, in some embodiments, comprise a display screen, a graphical user interface (GUI) or other visual rendering component (e.g. an augmented reality component, or a virtual reality component/headset), one or more speakers, one or more microphones or any other audio component, one or more lights, a component for providing tactile feedback (e.g. a vibration function), or any other user interface, or combination of user interfaces for providing information relating to, or resulting from the method, to the user. In some embodiments, the user interface 104 may be part of a device that also comprises one or more other components of the apparatus 100 (for example, the processor 102, the memory 106 and/or one or more other components of the apparatus 100). In alternative embodiments, the user interface 104 may be part of a separate device to the other components of the apparatus 100.

In some embodiments, as illustrated in Fig. 1, the apparatus 100 may also comprise a communications interface (or circuitry) 108 for enabling the apparatus 100 to communicate with any interfaces, memories and devices that are internal or external to the apparatus 100. The communications interface 108 may communicate with any interfaces, memories and devices wirelessly or via a wired connection.

It will be appreciated that Fig. 1 shows the components required to illustrate this aspect of the disclosure and, in a practical implementation, the apparatus 100 may comprise other components in addition to those shown. For example, the apparatus 100 may comprise a battery or other power supply for powering the apparatus 100 or means for connecting the apparatus 100 to a mains power supply.

As noted above, the processor 102 is configured to perform steps of the methods described herein. In some embodiments, a memory (e.g. the memory 106) may be configured to store a set of instructions which, when executed by the processor 102 of the apparatus 100, cause the processor 102 to perform steps or functions as discussed below. The processor 102 is configured to obtain a portion of verbal communication content to be presented to a user. The portion of verbal content to be presented to a user may comprise pre-scripted content, such as a word, a phrase, a sentence or a passage of text to be delivered to the user, for example audibly or visually. In some examples, the content may be delivered to user in some other format, such as Braille. In embodiments where the content is to be delivered to a user as part of a coaching session, such as a health management coaching session, pre-scripted content to be delivered may include commonly-used portions of dialogue, such as a greeting (e.g. "good morning, how are you feeling today?") or an information-gathering question or statement (e.g. "tell me about any exercise you have done today"). In general, most, if not all, of the content to be delivered to the user may comprise pre-scripted portions of content. For example, a portion of content to be delivered may be obtained from a database in a storage medium (e.g. the memory 106), or constructed from sub-portions of content (e.g. words or phrases) in a database. In some examples, sub-portions of verbal communication content may be combined with other sub-portions of verbal communication content to create a complete portion (e.g. a sentence) in response to a user input, such as a user's previous response to a question posed by the conversational agent. In some embodiments, one or more sub-portions of verbal content may be generated by and/or retrieved from an external system (not shown).

In some embodiments, different conversation content and portions and sub-portions of conversation content may be stored in different databases and/or different storage mediums depending on the intended use of the content. For example, a first database may store content relevant to a health management coaching scenario, and a second database may store content relevant to a customer service provision scenario.

In other examples, the portion of verbal communication content may be provided to the apparatus manually, for example by a health management coach. The content may be input via a user input interface (e.g. the user interface 104). In other examples, content may be obtained from a database and received via a user interface. Thus, the processor 102 may be configured to obtain the portion of verbal communication content from at least one of: a database; and a user input interface.

Content in the database(s) may be assigned to, or associated with, particular phases of a dialogue or communication exchange. For example, some content may be associated with an introduction phase, whereby such content may be used to begin or open a dialogue with the user; some content may be associated with an education phase, whereby such content may be used to inform or educate a user (e.g. to provide the user with a relevant fact regarding the importance of regular exercise); some content may be associated with a questioning phase, whereby such content may be delivered to the user in an attempt to obtain information (e.g. to find out whether the user has been exercising regularly); and a behavioral change phase, whereby such content may be used to prompt or motivate the user to change his or her behavior in some way (e.g. to convince the user to exercise more regularly). Content associated with other phases of a dialogue may also be included.

The processor 102 is further configured to provide the portion of verbal communication content as an input to an intent determination model. The intent determination model may be a machine learning model (e.g. a model trained using machine learning techniques), such as a classifier. Thus, the intent determination model may, in some examples, comprise a machine learning model trained to determine one or more intents from a portion of verbal communication content. The intent determination model may be executed within the apparatus 100 itself or remote from the apparatus, for example on a remote server using a cloud-computing arrangement.

The processor 102 is further configured to determine, using the intent determination model, at least one intent of the portion of verbal communication content. In other words, an output of the intent determination model may comprise one or more intents of the portion of verbal content. As noted above, an "intent" of a portion of the content can be considered to be the intended meaning of the communication or the purpose or goal expressed by the communication. For example, a non-exhaustive list of intents that might be associated with a portion of verbal content might include: agreeing, clarifying, educating, developing a discrepancy, elaborating, relating, educating, empathizing and sympathizing. Multiple intents may be obtained from a single statement, or portion of verbal content.

The determination of the at least one intent of the portion of verbal content by the intent determination model may be based at least on the verbal content itself (e.g. the words to be communicated and the order or structure of the words) and, in some embodiments, may also be based partly on other data and information, such as data relating to one or more users including, for example, the user (e.g. a patient) with whom the communication is to be made. Such additional data may include, for example, patient profiles for patient data from other sources, such as an electronic health record (EHR). Patient data may, in some examples, be used to determine topics of conversation (e.g. conversation content) to be included (e.g. referring to low-carb food when the patient data indicates that the patient is diabetic).

The intent determination model may be used to determine an intent or multiple intents for all of the portions of verbal content to be presented to a user during a dialogue (e.g. during a health management coaching session). Thus, each line of dialogue may have an associated intent (or multiple associated intents) determined by the intent determination model.

The processor 102 is further configured to determine, for the at least one intent, corresponding non-verbal communication content. In some examples, the corresponding non-verbal content may be determined by consulting a lookup table or database. Each intent may have corresponding non-verbal content associated with it, such as a particular facial expression, body language movement, gesture, tonal change and so on, and an indication of this non-verbal content may be stored in a database or lookup table in association with the corresponding intent. The non-verbal content may have one or more descriptors. For example, the intent "empathizing" may be associated with non-verbal communication content which has descriptors including a medium-to-high-pitched voice when the content is expressed audibly. When the content is delivered by an enabled conversational agent, such as an avatar displayed on a display device of a computer, then non-verbal communication content may further include tilting the head to one side, and/or displaying hands with palms facing upwards. Furthermore, the face of the avatar may be smiling. Other types of non-verbal content may also be associated with intents, including the "empathizing" intent. This may be referred to as non-verbal intent.

More generally, the non-verbal communication content may comprise at least one of: a visual focus of attention, or VFOA (e.g. an indication of where a subject is looking, such as health coach looking directly at the user or patient, health coach looking down, health coach making/not making eye contact), a facial expression (e.g. a motion or position of muscles beneath the skin of the face, causing expressions, such as a smile, a frown or a stern look, used to convey an emotional state, such as happy, sad, angry, focused, surprised, confused and so on), a head pose (e.g. nodding, head straight, head up or head tilted to one side), a physical movement (e.g. leaning forwards, reclining or sitting up straight), a posture (e.g. a position of the body while standing or sitting, such as a closed posture with arms crossed, clasped hands, an open posture with head up, head up with open hands or head up with arms folded), a gesture (e.g. a movement of part of the body to express an idea or feeling, such as a "calm down" gesture with palms of both hands facing down, palms of hands facing in an upwards position, elbows on the outside of a chair, thumbs up or clenching a fist), a mannerism (e.g. giggling, gasping or sighing), a voice modulation or inflection (e.g. increasing pitch, decreasing pitch or adjusting a volume), and a paralanguage element (e.g. an aspect of the voice including speed of speech, tone, volume or melody, and pauses and hesitations between words, including conversational speech at medium pitch or assertive speech at low pitch). In some examples, the non-verbal communication content may include the appearance of a face of an avatar (e.g. of the coach).

The processor 102 is further configured to associate the corresponding non-verbal communication content with the portion of verbal communication content. By associating the corresponding non-verbal content with the portion of verbal content, the portion of verbal content can be supplemented by the corresponding non-verbal content so that additional meaning and context may be included with the verbal content, thereby improving the effectiveness of the verbal content when it is delivered to a user. As described above, the processor 102 may, according to some embodiments, determine one or more intents of the verbal content, then associate the intent with corresponding non-verbal content. In other embodiments, the processor 102 may determine one or more intents for the verbal content and one or more intents for the non-verbal content, then associate the intents with one another.

After performing any of its steps or functions described above, the processor 102 may store or write an output of a step or function to a storage medium, such as the memory 106. For example, once at least one intent of the portion of verbal content has been determined, the at least one intent may be stored in a memory for retrieval at a later time. Thus, the processor 102 may, in some embodiments, store the association between the corresponding non-verbal communication content with the portion of verbal communication content in a storage medium. The processor 102 may, in some embodiments, store the association between the corresponding non-verbal communication content with the determined intent. The storage medium may, for example, comprise the memory 106. The stored association may then be retrieved and used when the same verbal content is to be delivered at a later time, for example to a different user. In this way, the processing (e.g. determining the intent and the corresponding non-verbal content) need not be repeated.

The processor 102 may, in some embodiments, be further configured to deliver the non-verbal communication content and the portion of verbal communication content for presentation to the user. In some embodiments, the content (i.e. verbal and non-verbal) may be transmitted (e.g. via a wired connection or wirelessly) to a device to be used for presenting the content to a user. In other examples, the apparatus 100 may present the content to the user itself, for example via the user interface 104. As noted above, the content may be presented or delivered to the user in a number of ways. In one example, the verbal content (e.g. a sentence, phrase or stream of words) may be displayed as text on a display screen viewable by the user. In this example, non-verbal content may be presented in the form of an image (e.g. a face representing the communicator of the content, such as a health management coach) exhibiting aspects of the non-verbal content. Alternatively or additionally, the non-verbal content may be presented audibly, for example with the verbal content spoken, with pitch changes and inflections in the voice representing the non-verbal content. In some examples, the non-verbal communication may be communicated to the user by a conversational agent. The conversational agent may have an "invisible" presence, such that the content being presented is visible to the user, but the conversational agent itself is not visible. In other examples, the verbal content may be presented by an embodied conversational agent, such as an avatar in a multi-modal system (i.e. a system using a combination of audio, video and/or text), representing the communicator (e.g. a health management coach). The embodied conversational agent may be an accurate representation of a person who might otherwise deliver the verbal content (e.g. a realistic representation of a health management coach), or a representation of a fictional being or character, such as a cartoon.

According to some embodiments disclosed herein, the verbal content analyzed to determine its intent is content spoken (or to be spoken) by a coach (e.g. a health management coach) or a representation thereof (e.g. a conversational agent). In other embodiments, however, at least on intent may be determined in respect of verbal content provided by the user (e.g. a patient). This may be used to decide which content is to be delivered by the coach next.

The determining of the at least one intent for the portion of verbal communication may be performed prior to a dialogue commencing between the apparatus (e.g. a conversational agent) and a user, or in real time, while a dialogue is taking place.

In some embodiments, the non-verbal communication content and the portion of verbal communication content to be presented to the user may be determined based on at least one previous interaction between the conversational agent and a user. For example, the processor 102 may select the next portion of verbal content to be presented to the user based on an earlier response (or other input) by that user, or based on a response (or other input) by any other user. In this way, if it has been determined that a particular statement or phrase to be presented is particularly effective (e.g. is particularly persuasive or motivational to the user) in response to a particular comment made by a user, then the same content may be presented when a user (i.e. the same user or a different user) makes the same particular comment at another time. Conversely, it may be the case that delivering certain verbal content to a user when the user makes a particular comment has a negative effect and, therefore, such content may be avoided if a user makes that particular comment at another time. This may be referred to as continuous learning, whereby the current trained model may be updated with additional information.

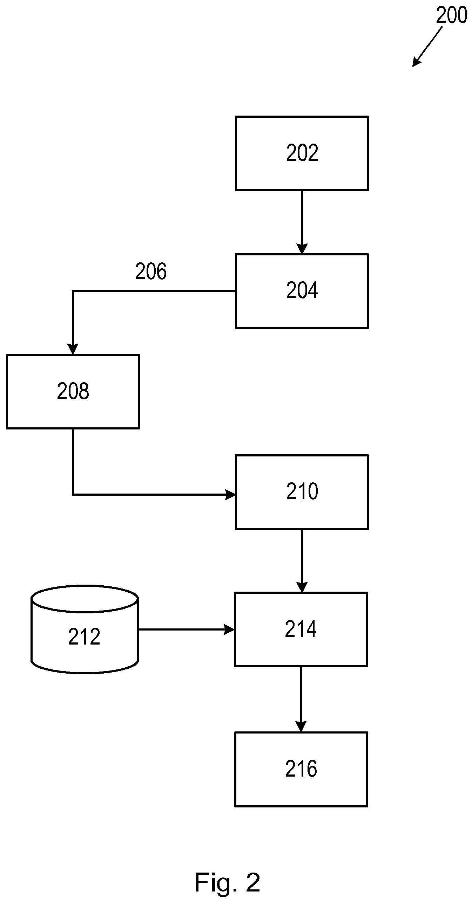

An example of a particular embodiment will now be described with reference to Fig. 2. In this example, content is to be delivered to a patient in the context of a health management coaching setting. Thus, the content may, for example, be delivered by a conversational agent posing as a health management coach. Fig. 2 is a flowchart of an example of a method 200 of associating non-verbal content with verbal content. At step 202 of the method 200, verbal communication content (e.g. a dialogue between a health management coach and a patient) to be used in an exchange with a user is obtained or received, for example from a database of content. The content may, for example, be pre-scripted content and/or may be based on previous conversations or dialogues held between two parties, such as a health management coach and the patient. In some embodiments, the verbal content may be annotated with phase annotations, which indicate the conversational phase in which each portion of content is intended to be used.

At step 204, the lines of the dialogue that are spoken by the health management coach are extracted. The extracted lines are provided (at 206) to an intent determination model 208 which, as discussed above, may comprise a machine learning model or classifier trained to determine a communicator's intent from their communications. At step 210, one or more intents of the extracted lines are determined, using the intent determination model 208. A database 212 containing a plurality of intents and corresponding non-verbal content is then accessed to determine non-verbal content for each of the intents extracted from the pre-scripted content (step 214). The determined non-verbal content may then be associated with the pre-scripted content, and this association may, for example, be stored in the database or memory. At step 216, the non-verbal content may be applied to the pre-scripted content, and presented to a user along with the pre-scripted content. For example, the pre-scripted dialogue of a health management coaching session may be delivered to a patient by an avatar displayed on a computing device display screen, and the associated non-verbal content may be delivered by applying the non-verbal content to the avatar, for example by adjusting the way in which the avatar presents the verbal content. While the verbal and non-verbal content may be presented to the user in a number of ways, as discussed above, presenting content using an embodied conversational agent, such as an avatar, may have particular benefits. For example, content, such as instructions or advice, delivered to a user by an avatar may be more persuasive than if it were delivered in purely textual form, as a patient may be more inclined to take such advice from a human (or a representation of a human). By applying non-verbal content to such a representation of the human, such as a health management coach, emotions may be expressed and/or displayed, which might have a further persuasive effect on the patient.

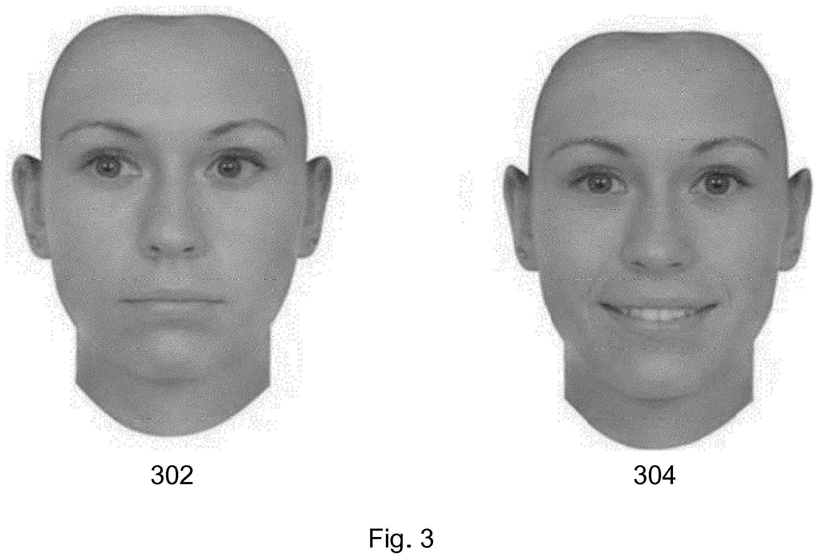

Various emotions may be expressed using non-verbal content. In examples where an avatar for representation of a human coach is used to deliver content to a user, facial expressions may be emulated on the avatar to convey emotions. For example, to convey happiness, an avatar's facial representation may be modified by raising the cheeks and lifting the corners of the lips. Fig. 3 is an example of an avatar that may, in some embodiments, be used to deliver content to a user. In Fig. 3, a neutral expression is shown on the face 302, and a happy expression is shown on the face 304. In other examples, other emotions may be conveyed. For example, to convey sadness, the avatar's brow may be raised, and the corners of the lips may be lowered. To convey disgust, the avatar's nose may be caused to wrinkle, the corners of the lips may be lowered, and the lower lip may be lowered.

So far, the disclosure has focused on how non-verbal content may be determined and associated with verbal content to be delivered to a user. Aspects of the invention also relate to training a model to determine intents from verbal content. The model may be trained using real-life data, such as recordings of actual health management coaching sessions between a human health coach and a patient. Thus, the processor 102 of the apparatus 100 may be further configured to perform functions relating to training a model, such as a classifier. Such steps may, for example, be performed prior to obtaining the portion of verbal content to be presented to the user. In some embodiments, the processor 102 may be configured to receive an annotated recording of an interaction between a first human (e.g. a health management coach) and a second human (e.g. a subject or patient receiving health management coaching), wherein the annotated recording comprises an indication of at least one intent associated with a portion of the interaction. The recording may, for example, comprise a video recording of the interaction which may, for example, form part of a health management coaching session, and may include both verbal content and non-verbal content. The recording may be annotated by one or more experts who have identified any intents that appear to be relevant for each portion of the interaction based on both the verbal aspects (words used) and the non-verbal aspects of the recording. A portion of the interaction may, for example, include a word, phrase, sentence, paragraph or other part of the interaction. The annotations may be made using any suitable techniques, such as storing each portion of the interaction as a separate data file, and providing annotations in the form of metadata to be stored with the data files. The annotated recording (e.g. in the form of one or more data files) may then be stored in a storage medium accessible by the processor 102.

The processor 102 may be further configured to train a classifier using the portion of the interaction and the at least one intent. The trained classifier may comprise the intent determination model. Thus, the portion of the interaction and the at least one intent may be provided as inputs for the classifier, so that the classifier can be trained to recognize intents from verbal content.

The processor 102 may be further configured to receive an indication of non-verbal communication content associated with the portion of the interaction. Again, an annotator (e.g. the human or humans who performed the annotation of the recording) may provide such an indication of non-verbal cues (e.g. facial expressions, gestures, movements, and so on) that are evident in each portion of the interaction. For example, an annotator may see that, during a particular portion of the interaction, the health coach laughs and smiles. Accordingly, the annotator may provide an indication that laughter and smiling constitute the non-verbal communication content for that particular portion of the interaction and, more particularly, for the particular intent identified by the annotators for that portion of the interaction. The indication of non-verbal content may be stored in a database in a storage medium, for example associated with the corresponding intent or intents.

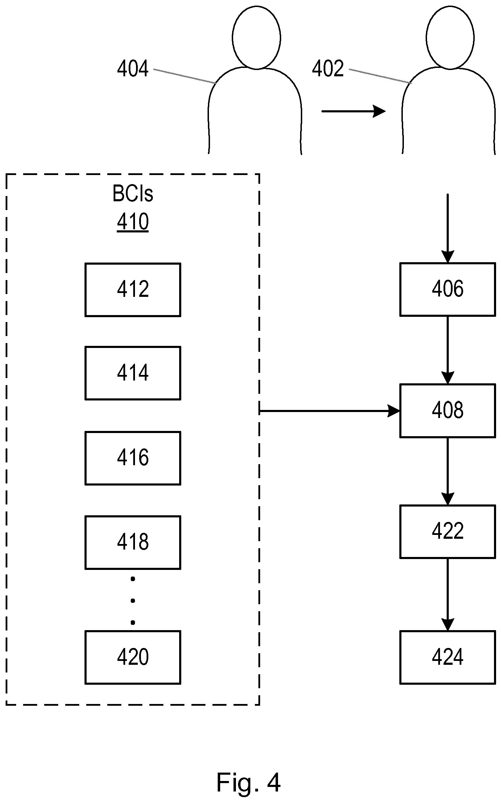

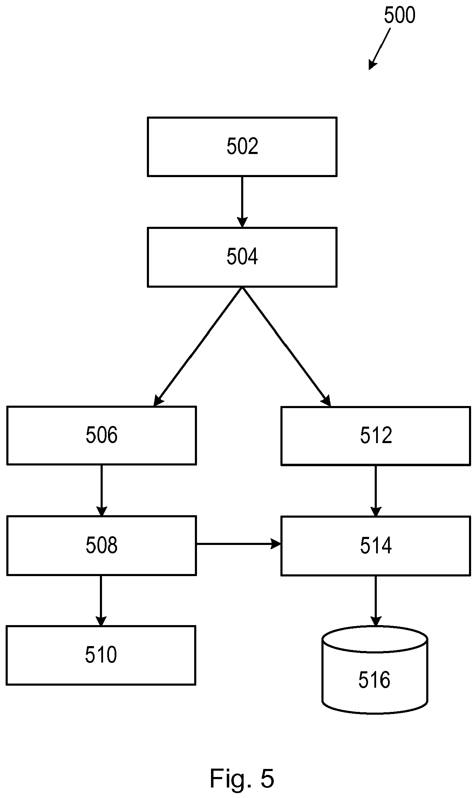

An example of a particular embodiment will now be described with reference to Figs. 4 and 5. In this example, content is again to be delivered to a patient in the context of a health management coaching setting. Referring to Fig. 4, a health management coach 402 takes part in a health management coaching session with a patient 404. At step 406, the health management coach 402 may study a patient profile or patient history associated with the patient 404 to understand more about the patient, to determine techniques that might be used to best coach the patient. Depending on the patient (e.g., amongst other things, their medical background, their personality and experiences) different behavioral change techniques may be implemented to achieve the intended effect with regard to the patient 404. For some patients, an appropriate technique leading to a change in behavior might be educating them and allowing the patient to decide for themselves to change a particular behavior. For other patients, a more direct, forceful technique may be more appropriate, which might, for example, require the health management coach to explain bluntly the consequences for the patient if they do not change their behavior. Thus, at step 408, the health management coach 402 may select a suitable technique (or multiple techniques) to be used during the coaching session to best achieve the intended outcome.

Techniques for changing the patient's behavior may be referred to as behavior change interventions (BCIs), and different coaching or therapeutic methods may be considered to comprise different 'ingredients', which may be combined to achieve a desired outcome. For example, journaling and cognitive restructuring are ingredients of a particular type of therapy, cognitive behavioral therapy. Other types of therapy include motivational interviewing, cognitive therapy, and rational emotive therapy. The technique and/or the ingredients to be used (selected at step 408) may be chosen from a set of BCIs 410. The BCIs 410 may be different for different methods or therapies. Ingredients 412 to 418 shown in Fig. 4 are examples of some types of behavior change interventions that may be selected for use by the coach 402. The first type of BCI is 'prompt intention formation' 412, which involves encouraging a patient to set a general goal or to make a behavioral resolution, such as "I will take more exercise next week". Prompt intention formation is intended to encourage patients to decide to change. A second type of BCI is 'prompt barrier identification' 414, which is intended to encourage a patient to consider potential barriers to behavioral changes, and plan ways to overcome them. A third type of BCI is 'setting graded tasks' 416, which involves setting easy-to-perform tasks for the patient, and making them increasingly difficult (but still achievable) until the intended behavior is performed. A fourth type of BCI is 'stress management' 418, which involves advising a patient about ways of reducing stress to facilitate performance of an intended behavior. A fifth type of BCI is 'roll with resistance' 420, which involves dealing with a patient's resistance to change their behavior in an effective manner. The resistance is not confronted directly, but rather reframed in a way that reduces the patient's resistance. The ellipsis shown in box 410 of Fig. 4 represents the possibility that the selection at box 408 could be made from many other BCI's 410.

Once a suitable behavioral change technique, and a suitable set of techniques has been selected (step 408), the coaching session between the health coach 402 and the patient 404 may be performed, at step 422. The coaching session performed at step 422 may be recorded (using a video camera) and, at step 424, the recorded session may be annotated with various data. The recorded session may be annotated with an indication of the type of behavior change intervention being implemented during the coaching session. The recorded session may further be annotated with an indication of a phase of the identified BCI. A coaching session may cover one or more phases of a single BCI having a particular treatment goal, or phases of several BCI's with multiple treatment goals. Phases of a BCI might include pre-contemplative interviewing, contemplative interviewing, preparatory dialogue, action-taking and maintenance. The recorded session may further be annotated with the identified intent or intents for each line of dialogue in the coaching session. As noted above, the annotations may be made by one or more humans, such as experts in the field of behavioral change.

The steps discussed above with reference to Fig. 4 may be performed in relation to multiple coaching sessions between many different coaches and patients. As will be discussed below, the recorded sessions and the corresponding annotations are used to train a model to function as the content determination model and, therefore, the training can be more effective if training data from a range of scenarios is used.

Referring now to Fig. 5, an example of an interaction pattern mining technique is described. At step 502, the recorded sessions that were annotated at step 424 are obtained, and at step 504, individual lines or portions of dialogue spoken by the health coach 402 or by the patient 404 are extracted from each recorded session. The individual lines or portions of dialogue, are then analyzed to determine the intents and any non-verbal content. Specifically, at step 506, the verbal content of the extracted lines or portions of dialogue are identified. The intent or intents of the verbal content is then determined at step 508. The determination of the intent(s) associated with the verbal content may be made by one or more humans (e.g. the expert or annotator who performed the annotation previously). Once the intents have been determined, the verbal content and associated intents are used, at step 510, to train a classifier, such as the intent recognition model. It will be appreciated that various different types of the classifier may be used, such as an artificial neural network, which will be familiar to those skilled in the art. In some examples, a separate classifier may be built or developed for each intent. In such examples, training data used to train each classifier may include a set of dialogue lines associated with a particular intent (i.e. positive labels) and a set of dialogue lines that are not associated with that particular intent (i.e. negative labels). Where multiple classifiers are used, each classifier may be applied separately, and the decision (i.e. the output) of one classifier may have no influence on the decision or output of any other classifier. In other examples, a single classifier may be developed to determine multiple intents from one or more lines of dialogue.

The individual lines and portions of dialogue extracted at step 504 are also analyzed to determine or extract, at step 512, non-verbal content (e.g. non-verbal cues) associated with each line portion of dialogue spoken by the health coach 402. At step 514, the non-verbal content is associated with the corresponding intents determined at step 508, and the association between the non-verbal content and the intents are stored in a database 516.

Table 1 below gives examples of lines of dialogue between a health coach and the patient, with the determined intents for each line of dialogue, and examples of non-verbal communication content identified when the dialogue lines are delivered.

**Table 1**

| Dialogue line | Intent(s) | Non-verbal communication content |
|---|---|---|
| Coach to patient: | Agreeing, clarifying, educating, developing discrepancy | VFOA: Coach looking at the patient |
| *"There are a few bad apples. Fortunately, that's pretty rare when you think about it. There are millions of different prescriptions. Most help, but there is always the potential for side effects.*" | | Head pose: Straight |
| | | Facial expressions: focused |
| | | Gesture: palms facing in an upward position |
| | | Paralanguage: conversational speech, medium pitch |
| | | Posture: head up, open hands |
| Patient to coach: | Elaborating | VFOA: Coach looking at the patient |
| *"I know. I looked at the insert from cold medicine the other day, and it was several inches long in tiny print. I couldn't even read it! Ridiculous!"* | | Head pose: nodding |
| | | Facial expressions: Light smile |
| | | Gesture: palms facing in an upward position |
| | | Paralanguage: |
| | | Posture: head up |
| Coach to patient: | Relating, educating, emphasizing | VFOA: Coach looking at the patient |
| *"I know! My pharmacist goes over side effects so it's clear before I take anything. Drug companies have to list virtually all potential side effects anybody anywhere might have - even when preexisting conditions are part of the issue. "* | | Head pose: Straight |
| | | Facial expressions: chuckling |
| | | Gesture: palms facing in an upward position |
| | | Paralanguage: conversational speech, medium pitch |
| | | Posture: head up, open hands |

Once the associations between the intents and the non-verbal content have been stored in the database 516, the database can be used to perform an association between non-verbal content and verbal content, for example as described above using the apparatus 100. Thus, the database 516 may be considered to be the same as the database 212.

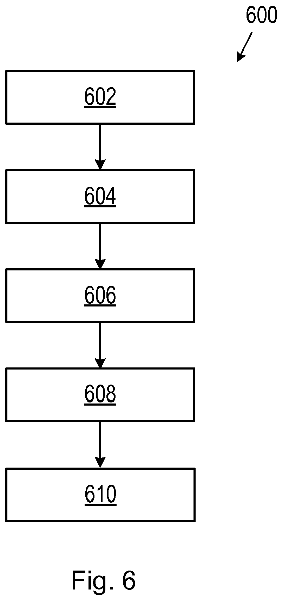

According to a second aspect, embodiments disclosed herein provide a method for associating non-verbal communication content with verbal communication content. Fig. 6 is a flowchart of an example of such a method 600. The method 600 comprises, at step 602, obtaining a portion of verbal communication content to be presented to a user. A portion of verbal communication content may, for example, comprise a pre-scripted portion of dialogue to be presented to a patient as part of a health coaching session. At step 604, the method 600 comprises providing the portion of verbal communication content as an input to an intent determination model. As noted above, the intent determination model may comprise a classifier trained using techniques disclosed herein, and may be trained using machine-learning techniques. The method 600 comprises, at step 606, determining, using the intent determination model, at least one intent of the portion of verbal communication content. Once an intent or intents have been determined for the portion of verbal content, the method 600 comprises, at step 608, determining, for the at least one intent, corresponding non-verbal communication content. For example, step 608 may include accessing a database, such as the database 212, 516, obtaining indication of non-verbal content that typically accompanies the at least one intent. At step 610, the method 600 comprises associating the corresponding non-verbal communication content with the portion of verbal communication content. Thus, by using the method 600, it is possible to start with a portion of verbal content, and determine some relevant non-verbal content to be used with the verbal content to enhance the effectiveness of the verbal content.

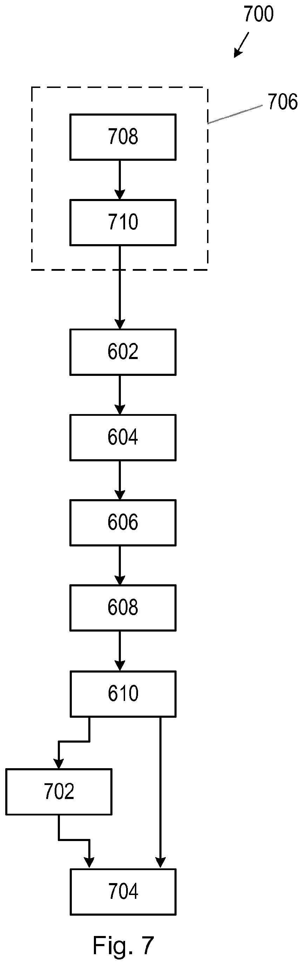

Fig. 7 is a flowchart of a further example of a method 700 for associating non-verbal communication content with verbal communication content. The method 700 may include steps of the method 600 discussed above. The method 700 may comprise, at step 702, storing the association between the corresponding non-verbal communication content with the portion of verbal communication content in a storage medium. For example, the association may be stored in a database, such as the database 212, 516. By storing the association, the association may be used again, for example when the same portion of verbal communication is used during a future coaching session. At step 704, the method 700 may comprise presenting the non-verbal communication content and the portion of verbal communication content to the user. For example, the verbal content may be presented to a user in the form of an avatar delivering the dialogue, and the non-verbal content may comprise features regarding how the verbal content is to be delivered, including, for example, the appearance of the avatar. Thus, the step of presenting (step 704) may comprise presenting the non-verbal communication content and the portion of verbal communication content to the user via a conversational agent. In some embodiments, presenting the non-verbal communication content via a conversational agent may comprise causing the conversational agent to exhibit non-verbal communication characteristics and/or non-verbal communication patterns based on the non-verbal communication content. The conversational agent may, in some examples, exhibit non-verbal content without exhibiting verbal content. This may be done, for example, while the patient is speaking. The steps 702 and 704 may be performed independently of one another, such that either step may be omitted.

Prior to performing this step 602, the method 700 may, in some embodiments, further comprise, at step 706, training an intent determination model. Training the intent determination model may comprise, at step 708, receiving an annotated recording of an interaction between a human coach and a subject, wherein the annotated recording comprises an indication of at least one intent associated with a portion of the interaction. Training the intent determination model may comprise, at step 710, training the intent determination model using the portion of the interaction and the at least one intent. As indicated in Fig. 7, once the intent determination model has been trained (step 710), the method may proceed to step 602.

Steps of the methods 600, 700 may, for example, be performed by the apparatus 100.

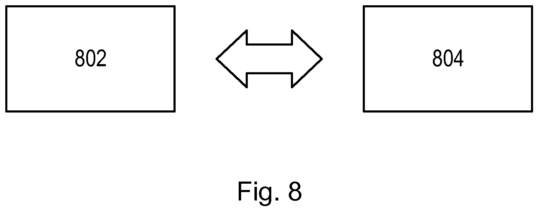

According to a third aspect, embodiments disclosed herein provide a computer program product. Fig. 8 is a simplified schematic of a processor 802 in communication with a computer-readable medium 804. According to some embodiments, a computer program product comprises a non-transitory computer-readable medium, the computer-readable medium 804 having computer-readable code embodied therein, the computer-readable code being configured such that, on execution by a suitable computer or processor 802, the computer or processor is caused to perform steps of the methods disclosed herein.

The processor 102, 802 can comprise one or more processors, processing units, multi-core processors or modules that are configured or programmed to control the apparatus 100 in the manner described herein. In particular implementations, the processor 102, 802 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for associating non-verbal communication content with verbal communication content, the apparatus comprising:
a processor (102) configured to:
obtain a portion of verbal communication content to be presented to a user;
provide the portion of verbal communication content as an input to an intent determination model;
determine, using the intent determination model, at least one intent of the portion of verbal communication content;
determine, for the at least one intent, corresponding non-verbal communication content; and
associate the corresponding non-verbal communication content with the portion of verbal communication content.

2. The apparatus (100) according to claim 1, wherein the processor (102) is further configured to:
store the association between the corresponding non-verbal communication content with the portion of verbal communication content in a storage medium.

3. The apparatus (100) according to claim 1 or claim 2, wherein the processor (102) is further configured to:
deliver the non-verbal communication content and the portion of verbal communication content for presentation to the user.

4. The apparatus (100) according to any of the preceding claims, wherein the non-verbal communication content is to be communicated to the user by a conversational agent.

5. The apparatus (100) according to any of claim 4, wherein the non-verbal communication content and the portion of verbal communication content to be presented to the user is determined based on at least one previous interaction between the conversational agent and a user.

6. The apparatus (100) according to any of the preceding claims, wherein the non-verbal communication content comprises at least one of: a visual focus of attention, a facial expression, a head pose, a physical movement, a posture, a gesture, a mannerism, a voice modulation or inflection, and a paralanguage element.

7. The apparatus (100) according to any of the preceding claims, wherein the intent determination model comprises a machine learning model trained to determine one or more intents from a portion of verbal communication content.

8. The apparatus (100) according to any of the preceding claims, wherein the processor (102) is configured to obtain the portion of verbal communication content from at least one of: a database; and a user input interface.

9. The apparatus (100) according to any of the preceding claims, wherein the processor (102) is further configured to:
receive an annotated recording of an interaction between a first human and a second human, wherein the annotated recording comprises an indication of at least one intent associated with a portion of the interaction; and
train a classifier using the portion of the interaction and the at least one intent, wherein the trained classifier comprises the intent determination model.

10. A method (600) for associating non-verbal communication content with verbal communication content, the method comprising:
obtaining (602) a portion of verbal communication content to be presented to a user;
providing (604) the portion of verbal communication content as an input to an intent determination model;
determining (606), using the intent determination model, at least one intent of the portion of verbal communication content;
determining (608), for the at least one intent, corresponding non-verbal communication content; and
associating (610) the corresponding non-verbal communication content with the portion of verbal communication content.

11. The method (600, 700) according to claim 10, further comprising, at least one of:
storing (702) the association between the corresponding non-verbal communication content with the portion of verbal communication content in a storage medium; and
presenting (704) the non-verbal communication content and the portion of verbal communication content to the user.

12. The method (600, 700) according to claim 10 or claim 11, wherein said presenting (704) comprises presenting the non-verbal communication content and the portion of verbal communication content to the user via a conversational agent.

13. The method (600, 700) according to claim 12, wherein presenting (704) the non-verbal communication content via a conversational agent comprises causing the conversational agent to exhibit non-verbal communication characteristics and/or non-verbal communication patterns based on the non-verbal communication content.

14. The method (600, 700) according to any of claims 10 to 13, further comprising, prior to said obtaining:
training (706) an intent determination model by:
receiving (708) an annotated recording of an interaction between a human coach and a subject, wherein the annotated recording comprises an indication of at least one intent associated with a portion of the interaction; and
training (710) the intent determination model using the portion of the interaction and the at least one intent.

15. A computer program product comprising a non-transitory computer-readable medium, the computer-readable medium (804) having computer-readable code embodied therein, the computer-readable code being configured such that, on execution by a suitable computer or processor (802), the computer or processor is caused to perform the method of any of claims 10 to 14.
